# FASCICULE DE BREVET EUROPEEN

(11) **EP 1 721 636 B1**
(45) Date de publication et mention de la délivrance du brevet: **09.09.2009**
(21) Numéro de dépôt: 06300411.3
(22) Date de dépôt: 26.04.2006
(51) Int. Cl.: A61Q 5/04, A61Q 5/06, A61K 8/81, A61K 8/41

(54) **Procédé de mise en forme semi-permanente des cheveux**
Verfahren zur semipermanenten Haarverformung
Process for the semi-permanent shaping of the hair

(30) Priorité: 29.04.2005 FR 0551134
(43) Date de publication de la demande: 15.11.2006
(73) Titulaire: L'ORÉAL, 75008 Paris (FR)
(72) Inventeur: Devin-Baudoin, Priscille, 92170, Vanves (FR)
(74) Mandataire: Catherine, Alain

(56) Documents cités:
- EP-A- 1 064 921
- EP-A- 1 506 768
- WO-A-20/05087186
- US-A- 4 660 580

## Description

L'invention se rapporte à un procédé de mise en forme des cheveux.

Deux grandes catégories de produits de mise en forme des cheveux sont généralement utilisées : les produits de coiffage et les produits de permanente.

Les produits de coiffage permettent une mise en forme non permanente des cheveux. Ils s'utilisent sur des cheveux mouillés ou secs avant la mise en forme à la main ou à l'aide d'une brosse ou d'un peigne.

Après leur application sur les cheveux et après séchage, ces produits durcissent de façon importante. Cela se traduit par un toucher corporisé et sec nécessaire au maintien et au volume de la coiffure.

Cependant, ces produits de coiffage s'éliminent par shampooing. Il faut donc les appliquer quotidiennement.

Les produits de permanente permettent une mise en forme durable de la chevelure.

Généralement, la technique utilisée pour obtenir une déformation permanente des cheveux consiste, dans un premier temps, à réaliser l'ouverture des liaisons disulfures -S-S- de la kératine (cystine) en appliquant sur les cheveux préalablement mis sous tension (bigoudis et autres moyens de mise sous tension) une composition réductrice (étape de réduction) puis, de préférence après avoir rincé la chevelure ainsi traitée, à reconstituer dans un second temps lesdites liaisons disulfures en appliquant sur les cheveux toujours sous tension une composition oxydante (étape d'oxydation, dite aussi de fixation) de façon à donner finalement aux cheveux la forme recherchée.

La nouvelle forme imposée aux cheveux par un traitement chimique tel que ci-dessus est durable dans le temps et résiste notamment à l'action des lavages à l'eau ou par shampooings.

Cependant, une telle technique ne donne pas entièrement satisfaction. En effet, cette technique est très efficace pour modifier la forme des cheveux, mais très dégradante pour les fibres capillaires.

Il existe donc un besoin de disposer d'un procédé rapide et simple à l'emploi, qui ne soit pas agressif pour le cheveu, et qui permette d'obtenir une bonne tenue de la coiffure, rémanente au shampooing, les cheveux présentant une qualité cosmétique satisfaisante, notamment en terme de douceur, de brillance et d'absence de toucher collant. En particulier la brillance et le toucher sont nettement améliorés par rapport à un produit de coiffage et le respect de l'intégrité de la fibre et de sa couleur sont nettement améliorées par rapport à un produit de permanente.

On connaît du document FR 2 245 339 un fixateur en deux parties à appliquer simultanément ou consécutivement, la première partie étant constituée par une solution comprenant un silicate alcalin ou un sel d'aluminium, la deuxième partie étant constituée par une solution contenant un polymère utilisé à pH acide ou alcalin. Par interaction entre ces deux solutions, se dépose sur les cheveux un précipité d'acide silicique ou d'hydrate d'aluminium.

On connaît également du document GB 2 025 228 une composition de traitement pour cheveux contenant un mélange de polymère cationique et de polymère anionique, les polymères cationique et anionique étant aptes à former un complexe précipitable sur cheveux en présence de sel de calcium.

Ces deux systèmes n'apportent pas une cosméticité, et/ou une durabilité suffisante de l'effet obtenu.

Les documents EP-A-1 064 921, US-A-4 660 580, EP-A-1 506 768 et WO 2005/087186 décrivent des procédés de fixation de la coiffure comprenant l'application de polymères fixants, solubilisés, anioniques ou amphotères et l'application de sels minéraux ou organiques. Les procédés de ces documents nécessitent une étape d'ouverture des liaisons disulfures de la kératine des cheveux par une composition réductrice et une étape de reconstitution desdites liaisons par une composition oxydante.

L'invention vise donc à fournir un nouveau procédé de mise en forme des cheveux.

Ainsi, l'invention a pour objet un procédé de mise en forme des cheveux, comprenant les étapes suivantes :
(a) application sur les cheveux d'une première composition comprenant, dans un milieu cosmétiquement acceptable, un ou plusieurs polymères fixants solubilisés, le ou lesdits polymères fixants consistant en un ou plusieurs polymères anioniques et/ou amphotères, cette application étant éventuellement suivie d'un temps de pose de ladite première composition,
(b) application sur les cheveux d'une deuxième composition comprenant, dans un milieu cosmétiquement acceptable, au moins 0,15%, en poids du poids total de ladite deuxième composition, d'un ou plusieurs sels minéraux et/ou organiques, cette application étant éventuellement suivie d'un temps de pose de ladite deuxième composition,
   l'étape b) étant effectuée avant ou après l'étape a), puis
(c) rinçage des cheveux,
   une étape de mise en forme des cheveux s'effectuant soit après l'application de la première composition de l'étape a), soit après l'application de la deuxième composition de l'étape b), et avant l'étape c).

Ce temps de pose de la première ou de la deuxième composition peut s'effectuer à température ambiante ou avec un apport de chaleur compris entre 30 et 250°, cet apport de chaleur pouvant être obtenu au moyen d'un sèche cheveux, d'un casque, d'un fer à lisser ou d'un fer à friser, ou par un dispositif générant de la vapeur, ou un dispositif générateur d'infrarouge.

On obtient ainsi une mise en forme des cheveux qui est rémanente à un ou plusieurs shampooings, de préférence à au moins deux shampooings.

Le procédé selon l'invention ne comprend pas d'étape d'ouverture des liaisons disulfures de la kératine des cheveux par une composition réductrice, ni d'étape de reconstitution desdites liaisons disulfures par une composition oxydante.

Par polymère fixant, on entend au sens de la présente invention tout polymère permettant de conférer une forme à la chevelure ou de modifier la forme de ladite chevelure.

Comme expliqué précédemment, le ou les polymères fixants consistent en un ou plusieurs polymères anioniques et/ou amphotères.

De préférence, la composition de l'étape (a) ne contient pas de polymère cationique.

Les polymères anioniques généralement utilisés dans la présente invention sont des polymères comportant des groupes dérivés d'acides carboxylique, sulfonique ou phosphorique, et présentant une masse moléculaire en poids comprise entre 500 et 5 000 000.

Les groupements carboxyliques sont apportés par des monomères monoacides ou diacides carboxyliques insaturés tels que ceux répondant à la formule : 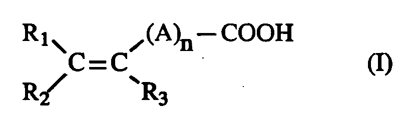 dans laquelle n est un nombre entier de 0 à 10, A désigne un groupement méthylène, éventuellement relié à l'atome de carbone du groupement insaturé ou au groupement méthylène voisin lorsque n est supérieur à 1, par l'intermédiaire d'un hétéroatome tel que l'oxygène ou le soufre, R₁ désigne un atome d'hydrogène, un groupement phényle ou benzyle, R₂ désigne un atome d'hydrogène, un groupement alkyle inférieur ou carboxyle, R₃ désigne un atome d'hydrogène, un groupement alkyle inférieur, un groupement -CH₂₋COOH, phényle ou benzyle.

Dans la formule (I) ci-dessus, un groupement alkyle inférieur comporte de préférence de 1 à 4 atomes de carbone et désigne en particulier, les groupements méthyle et éthyle.

Les polymères anioniques à groupements carboxyliques ou sulfoniques préférés selon l'invention sont :
A) les homo- ou copolymères d'acide acrylique ou méthacrylique ou leurs sels, dont les copolymères d'acide acrylique et d'acrylamide et les copolymères acide méthacrylique/acide acrylique/acrylate d'éthyle/méthacrylate de méthyle, en particulier l'AMERHOLD DR 25 commercialisé par la société AMERCHOL, et les sels de sodium des acides polyhydroxycarboxyliques. On peut citer également les copolymères acide méthacrylique/acrylate d'éthyle, notamment en dispersion aqueuse, tels que les LUVIFLEX SOFT et LUVIMER MAE commercialisés par la société BASF.
B) Les copolymères des acides acrylique ou méthacrylique avec un monomère monoéthylénique tel que l'éthylène, le styrène, les esters vinyliques, les esters d'acide acrylique ou méthacrylique, éventuellement greffés sur un polyalkylène-glycol tel que le polyéthylène-glycol, et éventuellement réticulés. De tels polymères sont décrits en particulier dans le brevet français 1 222 944 et la demande allemande n° 2 330 956, les copolymères de ce type comportant dans leur chaîne un motif acrylamide éventuellement N-alkylé et/ou hydroxyalkylé tels que décrits notamment dans les demandes de brevets luxembourgeois 75370 et 75371. On peut également citer les copolymères d'acide acrylique et de méthacrylate d'alkyle en C₁-C₄.
C) les copolymères dérivés d'acide crotonique tels que ceux comportant dans leur chaîne des motifs acétate ou propionate de vinyle, et éventuellement d'autres monomères tels que les ester allylique ou méthallylique, éther vinylique ou ester vinylique d'un acide carboxylique saturé, linéaire ou ramifié, à longue chaîne hydrocarbonée, tels que ceux comportant au moins 5 atomes de carbone, ces polymères pouvant éventuellement être greffés et réticulés, ou encore un ester vinylique, allylique ou méthallylique d'un acide carboxylique α ou β-cyclique. De tels polymères sont décrits entre autres dans les brevets français numéros 1 222 944, 1 580 545, 2 265 782, 2 265 781, 1 564 110 et 2 439 798. Des produits commerciaux entrant dans cette classe sont les résines 28-29-30, 26-13-14 et 28-13-10 vendues par la société NATIONAL STARCH.
   On peut citer aussi comme copolymère dérivé d'acide crotonique les terpolymères acide crotonique/acétate de vinyle/ t.butylbenzoate de vinyle et en particulier le MEXOMERE PW fourni par la société CHIMEX.
D) Les polymères dérivés d'acides ou d'anhydrides maléique, fumarique, itaconique avec des esters vinyliques, des éthers vinyliques, des halogénures vinyliques, des dérivés phénylvinyliques, l'acide acrylique et ses esters ; ces polymères peuvent être estérifiés. De tels polymères sont décrits en particulier dans les brevets US 2 047 398, 2 723 248, 2 102 113, le brevet GB 839 805, et notamment ceux vendus sous les dénominations GANTREZ® AN ou ES par la société ISP.
   Des polymères entrant également dans cette classe sont les copolymères d'anhydrides maléique, citraconique, itaconique et d'un ester allylique ou méthallylique comportant éventuellement un groupement acrylamide, méthacrylamide, une α-oléfine, des esters acryliques ou méthacryliques, des acides acryliques ou méthacryliques ou la vinylpyrrolidone dans leur chaîne, les fonctions anhydrides sont monoestérifiées ou monoamidifiées. Ces polymères sont par exemple décrits dans les brevets français 2 350 384 et 2 357 241 de la demanderesse.
E) Les polyacrylamides comportant des groupements carboxylates.
F) Les polymères comprenant les groupements sulfoniques. Ces polymères peuvent être des polymères comportant des motifs vinylsulfonique, styrène-sulfonique, naphtalène-sulfonique, acrylamidoalkylsulfonique, sulfoisophtalates.
   Ces polymères peuvent être notamment choisis parmi :
   - les sels de l'acide polyvinylsulfonique ayant une masse moléculaire comprise entre environ 1 000 et 100 000 ainsi que les copolymères avec un comonomère insaturé tel que les acides acrylique ou méthacrylique, et leurs esters, ainsi que l'acrylamide ou ses dérivés, les éthers vinyliques et la vinylpyrrolidone ;
   - les sels de l'acide polystyrène-sulfonique, les sels de sodium, ayant une masse moléculaire d'environ 500 000 et d'environ 100 000. Ces composés sont décrits dans le brevet FR 2198719 ;
   - les sels d'acides polyacrylamide-sulfoniques tels que ceux mentionnés dans le brevet US 4 128 631 ;
G) les polymères siliconés anioniques greffés ;

Les polymères siliconés greffés utilisés selon l'invention sont choisis préférentiellement parmi les polymères à squelette organique non-siliconé greffé par des monomères contenant un polysiloxane, les polymères à squelette polysiloxanique greffé par des monomères organiques non-siliconés et leurs mélanges.

Dans tout ce qui suit ou qui précède, on entend désigner par silicone ou polysiloxane, en conformité avec l'acceptation générale, tout polymère oligomère organosilicié à structure linéaire ou cyclique, ramifiée ou réticulée, de poids moléculaire variable, obtenus par polymérisation et/ou polycondensation de silanes convenablement fonctionnalisés, et constitués pour l'essentiel par une répétition de motifs principaux dans lesquels les atomes de silicium sont reliés entre eux par des atomes d'oxygène (liaison siloxane ≡Si-O-Si≡), des radicaux hydrocarbonés éventuellement substitués étant directement liés par l'intermédiaire d'un atome de carbone sur lesdits atomes de silicium. Les radicaux hydrocarbonés les plus courants sont les radicaux alkyles notamment en C₁-C₁₀ et en particulier méthyle, les radicaux fluoralkyles, les radicaux aryles et en particulier phényle, et les radicaux alcényles et en particulier vinyle ; d'autres types de radicaux susceptibles d'être liés soit directement, soit par l'intermédiaire d'un radical hydrocarboné, à la chaîne siloxanique sont notamment l'hydrogène, les halogènes et en particulier le chlore, le brome ou le fluor, les thiols, les radicaux alcoxy, les radicaux polyoxyalkylènes (ou polyéthers) et en particulier polyoxyéthylène et/ou polyoxypropylène, les radicaux hydroxyles ou hydroxyalkyles, les groupements aminés substitués ou non, les groupements amides, les radicaux acyloxy ou acyloxyalkyles, les radicaux hydroxyalkylamino ou aminoalkyles, des groupements ammonium quaternaires, des groupements amphotères ou bétaïniques, des groupements anioniques tels que carboxylates, thioglycolates, sulfosuccinates, thiosulfates, phosphates et sulfates, cette liste n'étant bien entendu nullement limitative (silicones dites « organomodifiées »).

Dans ce qui suit, on entend désigner par « macromère polysiloxane » en conformité avec l'acceptation générale, tout monomère contenant dans sa structure une chaîne polymère du type polysiloxane.

Les polymères à squelette organique non-siliconé greffé par des monomères contenant un polysiloxane utilisé selon la présente invention sont constitués d'une chaîne principale organique formée à partir de monomères organiques ne comportant pas de silicone, sur laquelle se trouve greffé, à l'intérieur de ladite chaîne ainsi qu'éventuellement à l'une au moins de ses extrémités, au moins un macromère polysiloxane.

Les monomères organiques non-siliconés constituant la chaîne principale du polymère siliconé greffé peuvent être choisis parmi des monomères à insaturation éthylénique polymérisables par voie radicalaire, des monomères polymérisables par polycondensation tels que ceux formant des polyamides, des polyesters, des polyuréthanes, des monomères à ouverture de cycle tels que ceux du type oxazoline ou caprolactone.

Les polymères à squelette organique non-siliconé greffé par des monomères contenant un polysiloxane conformes à la présente invention peuvent être obtenus selon tout moyen connu de l'homme de l'art, en particulier par réaction entre (i) un macromère polysiloxane de départ correctement fonctionnalisé sur la chaîne polysiloxanique et (ii) un ou plusieurs composés organiques non-siliconés, eux-mêmes correctement fonctionnalisés par une fonction qui est capable de venir réagir avec le ou les groupements fonctionnels portés par ladite silicone en formant une liaison covalente ; un exemple classique d'une telle réaction est la réaction radicalaire entre un groupement vinylique porté sur une des extrémités de la silicone avec une double liaison d'un monomère à insaturation éthylénique de la chaîne principale.

Les polymères à squelette organique non-siliconé greffé par des monomères contenant un polysiloxane, conformes à l'invention, sont choisis plus préférentiellement parmi ceux décrits dans les brevets US 4,693,935, US 4,728,571 et US 4,972,037 et les demandes de brevet EP-A-0412704, EP-A-0412707, EP-A-0640105 et WO 95/00578. Il s'agit de copolymères obtenus par polymérisation radicalaire à partir de monomères à insaturation éthylénique et de macromères siliconés ayant un groupe vinylique terminal
ou bien des copolymères obtenus par réaction d'une polyoléfine comprenant des groupes fonctionnalisés et d'un macromère polysiloxane ayant une fonction terminale réactive avec lesdits groupes fonctionnalisés.

Une famille particulière de polymères siliconés greffés convenant pour la réalisation de la présente invention est constituée par les copolymères greffés siliconés comprenant :
a) de 0 à 98 % en poids d'au moins un monomère (A) lipophile de faible polarité lipophile à insaturation éthylénique, polymérisable par voie radicalaire ;
b) de 1 à 98 % en poids d'au moins un monomère (B) hydrophile polaire à insaturation éthylénique, copolymérisable avec le ou les monomères du type (A) ;
c) de 0,01 à 50 % en poids d'au moins un macromère polysiloxane (C) de formule générale :

   X(Y)ₙSi(R)₃₋ₘZₘ (II)

   Où:
   X désigne un groupe vinylique copolymérisable avec les monomères (A) et (B) ;
   Y désigne un groupe de liaison divalent ;
   R désigne un hydrogène, un alkyle ou un alcoxy en C₁-C₆, un aryle C₆-C₁₂;
   Z désigne un motif polysiloxane monovalent ayant un poids moléculaire moyen en nombre d'au moins 500 ;
   n est 0 ou 1 et m est un entier allant de 1 à 3 ; les pourcentages étant calculés par rapport au poids total des monomères (A), (B) et (C).

Ces polymères sont décrits ainsi que leurs procédés de préparation dans les brevets US 4,963,935, US 4,728,571 et US 4,972,037 et les demandes de brevet EP-A-0412704, EP-A-0412707, EP-A-0640105. Ils ont un poids moléculaire moyen en nombre de préférence allant de 10 000 à 2 000 000 et de préférence une température de transition vitreuse Tg ou une température de fusion cristalline Tm d'au moins -20°C.

On peut citer comme exemples de monomères lipophiles (A), les esters d'acide acrylique ou méthacrylique d'alcools en C₁-C₁₈ le styrène ; les macromères polystyrène ; l'acétate de vinyle; le propionate de vinyle; l'alpha-méthylstyrène ; le tertio-butylstyrène ; le butadiène ; le cyclohexadiène ; l'éthylène ; le propylène ; le vinyltoluène ; les esters d'acide acrylique ou méthacrylique et de 1,1-dihydroperfluoroalcanol ou de ses homologues ; les esters d'acide acrylique ou méthacrylique et de oméga-hydrydofluoroalcanol ; les esters d'acide acrylique ou méthacrylique et de fluoroalkylsulfoamido-alcool ; les esters d'acide acrylique ou méthacrylique et d'alcool fluoroalkylique ; les esters d'acide acrylique ou méthacrylique et de fluoréther d'alcool ; ou leurs mélanges.

Les monomères (A) préférentiels sont choisis dans le groupe constitué par le méthacrylate de n-butyle, le méthacrylate d'isobutyle, l'acrylate de tertio-butyle, le méthacrylate de tertio-butyle, le méthacrylate de 2-éthylhexyle, le méthacrylate de méthyle, le 2-(N-méthyl perfluoroctane sulfoamido)éthylacrylate ; le 2-(N-butylperflurooctane sulfoamido)éthylacrylate et leurs mélanges.

On peut citer comme exemples de monomères polaires (B), l'acide acrylique, l'acide méthacrylique, le N,N-diméthylacrylamide, le méthacrylate de diméthylaminoéthyle, le méthacrylate de diméthylaminoéthyle quaternisé, le (méth)acrylamide, le N-t-butylacrylamide, l'acide maléique, l'anhydride maléique et leurs demi-esters, les (méth)acrylates hydroxyalkylés, le chlorure de diallyldiméthylammonium, la vinylpyrrolidone, les éthers de vinyle, les maléimides, la vinylpyridine, le vinylimidazole, les composés polaires vinyliques hétérocycliques, le styrène sulfonate, l'alcool allylique, l'alcool vinylique, le vinylcaprolactame ou leurs mélanges. Les monomères (B) préférentiels sont choisis dans le groupe constitué par l'acide acrylique, le N,N-diméthylacrylamide, le méthacrylate de diméthylaminoéthyle, le méthacrylate de diméthylaminoéthyle quaternisé, la vinylpyrrolidone et leurs mélanges.

A titre de monomères polaires (B), les polymères siliconés greffés anioniques utilisés selon l'invention contiennent au moins un monomère anionique.

Les macromères polysiloxane (C) de formule (I) préférentiels sont choisis parmi ceux répondant à la formule générale suivante (III) : 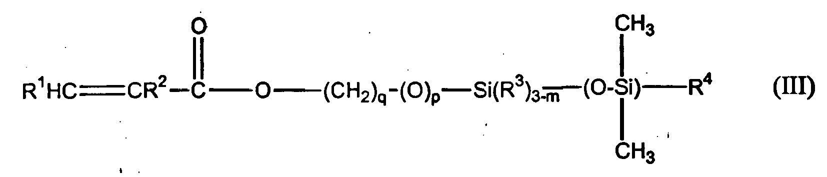 dans laquelle :
R¹ est l'hydrogène ou -COOH (de préférence hydrogène) ;
R² est l'hydrogène, méthyle ou -CH2COOH (de préférence méthyle) ;
R³ est un groupe alkyle, alcoxy ou alkylamino en C₁-C₆, aryle en C₁-C₁₂ ou hydroxyle (de préférence méthyle) ;
R⁴ est un groupe alkyle, alcoxy ou alkylamino en C₁-C₆, aryle en C₁-C₁₂ ou hydroxyle (de préférence méthyle) ;
q est un entier de 2 à 6 (de préférence 3) ;
p est 0 ou 1 ;
r est un nombre entier de 5 à 700 ;
m est un entier allant de 1 à 3 (de préférence 1);

On utilise plus particulièrement les macromères polysiloxanes de formule : 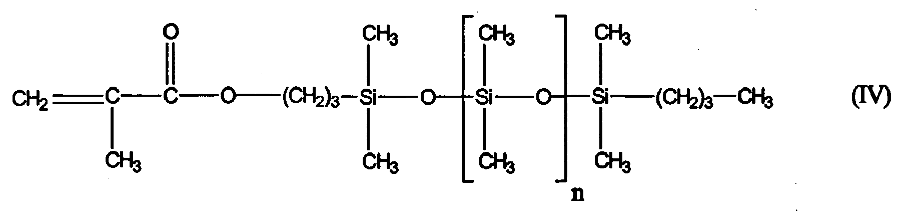 n étant un entier allant de 5 à 700.

Un mode particulier de réalisation de l'invention consiste à utiliser un copolymère susceptible d'être obtenu par polymérisation radicalaire à partir du mélange de monomères constitué par :
a) 60 % en poids d'acrylate de tertiobutyle :
b) 20 % en poids d'acide acrylique ;
c) 20 % en poids de macromère siliconé de formule : 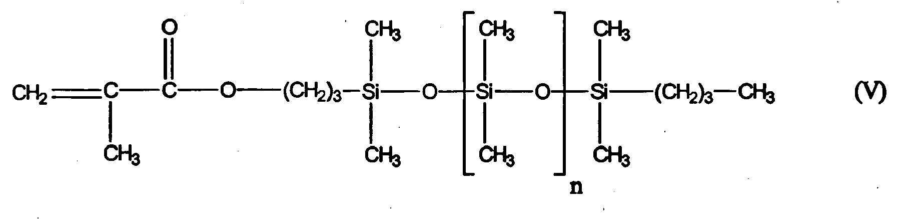
n étant un entier allant de 5 à 700 ; les pourcentages en poids étant calculés par rapport au poids total des monomères.

Une autre famille particulière de polymères siliconés convenant pour la réalisation de la présente invention est constituée par les copolymères greffés siliconés susceptibles d'être obtenus par extrusion réactive d'un macromère polysiloxane ayant une fonction réactive terminale sur un polymère du type polyoléfine comportant des groupes réactifs susceptibles de réagir avec la fonction terminale du macromère polysiloxane pour former une liaison covalente permettant le greffage de la silicone sur la chaîne principale de la polyoléfine.

Ces polymères sont décrits ainsi que leur procédé de préparation dans la demande de brevet WO 95/00578.

Les polyoléfines réactives sont choisies de préférence parmi les polyéthylènes ou les polymères de monomères dérivés de l'éthylène tels que le propylène, le styrène, les alkylstyrènes, le butylène, le butadiène, les (méth)acrylates, les esters de vinyle ou équivalents, comportant des fonctions réactives susceptibles de réagir avec la fonction terminale du macromère polysiloxane. Ils sont choisis plus particulièrement parmi les copolymères d'éthylène ou de dérivés d'éthylène et de monomères choisis parmi ceux comportant une fonction carboxylique tels que l'acide (méth)acrylique ; ceux comportant une fonction anhydride d'acide tels que l'anhydride de l'acide maléique ; ceux comportant une fonction chlorure d'acide tels que le chlorure de l'acide (méth)acrylique ; ceux comportant une fonction ester tels que les esters de l'acide (méth)acrylique ; ceux comportant une fonction isocyanate.

Les macromères siliconés sont choisis de préférence parmi les polysiloxanes comportant un groupe fonctionnalisé, en bout de la chaîne polysiloxanique ou à proximité de l'extrémité de ladite chaîne, choisi dans le groupe constitué par les alcools, les thiols, les époxy, les amines primaires et secondaires et plus particulièrement parmi ceux répondant à la formule générale (VI) :

T-(CH₂)ₛ-Si-[(OSiR⁵R⁶)ₜ-R⁷]_{y} (VI)

dans laquelle T est choisi dans le groupe constitué par NH₂, NHR', une fonction époxy, OH, SH ; R⁵, R⁶, R⁷ et R', indépendamment, désignent un alkyle en C₁-C₆, phényle, benzyle, ou alkylphényle en en C₆-C₁₂, hydrogène ; s est un nombre de 2 à 100 ; t est un nombre de 0 à 1000 et y est un nombre de 1 à 3. Ils ont un poids moléculaire moyen en nombre de préférence allant de 5 000 à 300 000, plus préférentiellement de 8 000 à 200 000 et plus particulièrement de 9 000 à 40 000.

Selon la présente invention, le ou les polymères siliconés greffés, à squelette polysiloxanique greffé par des monomères organiques non-siliconés comprennent une chaîne principale de silicone (ou polysiloxane (≡Si-O-)ₙ) sur laquelle se trouve greffé, à l'intérieur de ladite chaîne ainsi qu'éventuellement à l'une au moins de ses extrémités, au moins un groupement organique ne comportant pas de silicone.

Les polymères à squelette polysiloxanique greffé par des monomères organiques non-siliconés selon l'invention peuvent être des produits commerciaux existants, ou encore être obtenus selon tout moyen connu de l'homme de l'art, en particulier par réaction entre (i) une silicone de départ correctement fonctionnalisée sur un ou plusieurs de ces atomes de silicium et (ii) un composé organique non-siliconé lui-même correctement fonctionnalisé par une fonction qui est capable de venir réagir avec le ou les groupements fonctionnels portés par ladite silicone en formant une liaison covalente ; un exemple classique d'une telle réaction est la réaction d'hydrosylilation entre des groupements ≡Si-H et des groupements vinyliques CH₂=CH-, ou encore la réaction entre des groupements thio-fonctionnels-SH avec ces mêmes groupements vinyliques.

Des exemples de polymères à squelette polysiloxanique greffé par des monomères organiques non-siliconés convenant à la mise en oeuvre de la présente invention, ainsi que leur mode particulier de préparation, sont notamment décrits dans les demandes de brevets EP-A-0582152, WO 93/23009 et WO 95/03776 dont les enseignements sont totalement inclus dans la présente description à titre de références non limitatives.

Selon un mode particulièrement préféré de réalisation de la présente invention, le polymère siliconé, à squelette polysiloxanique greffé par des monomères organiques non-siliconés, mis en oeuvre comprend le résultat de la copolymérisation radicalaire entre d'une part au moins un monomère organique anionique non-siliconé présentant une insaturation éthylénique et/ou un monomère organique hydrophobe non-siliconé présentant une insaturation éthylénique et d'autre part une silicone présentant dans sa chaîne au moins un groupement fonctionnel capable de venir réagir sur lesdites insaturations éthyléniques desdits monomères non-siliconés en formant une liaison covalente, en particulier des groupements thio-fonctionnels.

Selon la présente invention, lesdits monomères anioniques à insaturation éthylénique sont de préférence choisis, seuls ou en mélanges, parmi les acides carboxyliques insaturés, linéaires ou ramifiés, éventuellement partiellement ou totalement neutralisés sous la forme d'un sel, ce ou ces acides carboxyliques insaturés pouvant être plus particulièrement l'acide acrylique, l'acide méthacrylique, l'acide maléique, l'anhydride maléique, l'acide itaconique, l'acide fumarique et l'acide crotonique. Les sels convenables sont notamment les sels d'alcalins, d'alcalino-terreux et d'ammonium. On notera que, de même, dans le polymère siliconé greffé final, le groupement organique à caractère anionique qui comprend le résultat de l'(homo)polymérisation radicalaire d'au moins un monomère anionique de type acide carboxylique insaturé peut être, après réaction, post-neutralisé avec une base (soude, ammoniaque,...) pour l'amener sous forme d'un sel.

Selon la présente invention, les monomères hydrophobes à insaturation éthylénique sont de préférence choisis, seuls ou en mélanges, parmi les esters d'acide acrylique alcanols et/ou les esters d'acide méthacrylique d'alcanols. Les alcanols sont de préférence en C₁-C₁₈ et plus particulièrement en C₁-C₁₂. Les monomères préférentiels sont choisis dans le groupe constitué par le (méth)acrylate d'isooctyle, le (méth)acrylate d'isononyle, le 2-éthylhexyl(méth)acrylate, le (méth)acrylate de lauryle, le (méth)acrylate d'isopentyle, le (méth)acrylate de n-butyle, le (méth)acrylate d'isobutyle, le (méth)acrylate de méthyle, le (méth)acrylate de tertio-butyle, le (méth)acrylate de tridécyle, le (méth)acrylate de stéaryle ou leurs mélanges.

Une famille de polymères siliconés à squelette polysiloxanique greffé par des monomères organiques non-siliconés convenant particulièrement bien à la mise en oeuvre de la présente invention est constituée par les polymères siliconés comportant dans leur structure le motif de formule (VII) suivant : 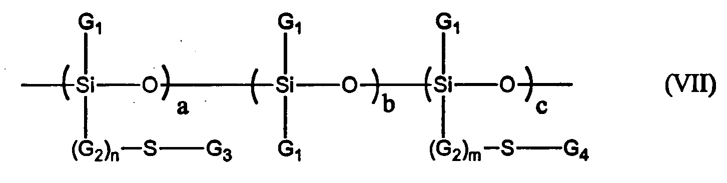 dans lequel les radicaux G₁, identiques ou différents, représentent l'hydrogène ou un radical alkyle en C₁-C₁₀ ou encore un radical phényle ; les radicaux G₂, identiques ou différents, représentent un groupe alkyle en C₁₋C₁₀ ; G₃ représente un reste polymérique résultant de l'(homo)polymérisation d'au moins un monomère anionique à insaturation éthylénique ; G₄ représente un reste polymérique résultant de l'(homo)polymérisation d'au moins un monomère hydrophobe à insaturation éthylénique ; m et n sont égaux à 0 ou 1 ; a est un nombre entier allant de 0 et 50 ; b est un nombre entier pouvant être compris entre 10 et 350, c est un nombre entier allant de 0 et 50 ; sous réserve que l'un des paramètres a et c soit différent de 0.

De préférence, le motif de formule (IV) ci-dessus présente au moins l'une, et encore plus préférentiellement l'ensemble, des caractéristiques suivantes :
- les radicaux G₁ désignent un radical alkyle, de préférence le radical méthyle ;
- n est non nul, et les radicaux G₂ représentent un radical divalent en C₁-C₃, de préférence un radical propylène ;
- G₃ représente un radical polymérique résultant de l'(homo)polymérisation d'au moins un monomère du type acide carboxylique à insaturation éthylénique, de préférence l'acide acrylique et/ou l'acide méthacrylique ;
- G₄ représente un radical polymérique résultant de l'(homo)polymérisation d'au moins un monomère du type (méth)acrylate d'alkyle(C₁-C₁₀), de préférence le (méth)acrylate d'isobutyle ou de méthyle.

Des exemples de polymères siliconés greffés répondant à la formule (IV) sont notamment des polydiméthylsiloxanes (PDMS) sur lesquels sont greffés, par l'intermédiaire d'un chaînon de raccordement de type thiopropylène, des motifs polymères mixtes du type acide poly(méth)acrylique et du type poly(méth)acrylate de méthyle.

De préférence, la masse moléculaire en nombre des polymères siliconés à squelette polysiloxanique greffé par des monomères organiques non-siliconés de l'invention varie de 10 000 à 1 000 000 environ, et encore plus préférentiellement de 10 000 à 100 000 environ.

A titre de polymères siliconés greffés utilisables selon la présente invention, on peut citer le produit commercialisé par la société 3M sous la référence VS80.

### H) Les polyuréthanes anioniques.

Les polyuréthanes utilisés de préférence selon l'invention présentent de préférence un motif répétitif de base répondant à la formule (VIII) suivante :

-X'-B-X'-CO-NH-R-NH-CO- (VIII)

dans laquelle
- X' représente O et/ou NH,
- B est un radical hydrocarboné divalent, ce radical étant substitué ou non, et
- R est un radical divalent choisi parmi les radicaux alkylènes ramifiés ou non de type aromatique en C₆-C₂₀, aliphatique en C₁ à C₂₀, de préférence en C₁-C₆, cycloaliphatique en C₁ à C₂, de préférence en C₁-C₆, ces radicaux étant non substitués ou substitués par un ou plusieurs groupements halogène, alcoxy en C₁-C₄, aryle en C₆-C₃₀ en particulier phényle.

De préférence, le radical B est un radical divalent en C₁-C₃₀, de préférence C₂-C₁₀ et est porteur d'un groupement présentant une ou des fonctions carboxyliques et/ou une ou des fonctions sulfoniques, lesdites fonctions carboxyliques et/ou sulfoniques étant sous forme libre ou partiellement ou totalement neutralisées par une base minérale ou organique telle que les hydroxydes des métaux alcalins ou alcalinoterreux, l'ammoniaque et les alkylamines ou alcanolamines, les acides aminés organiques. De préférence B est le radical divalent issu de l'acide diméthylolpropionique.

Le radical R est avantageusement choisi parmi les radicaux répondant aux formules suivantes : 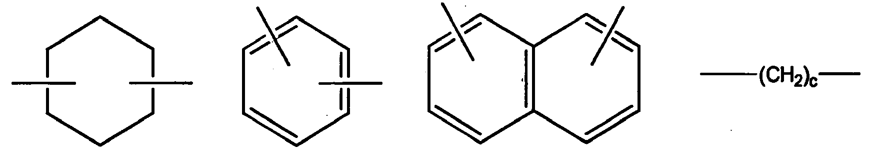 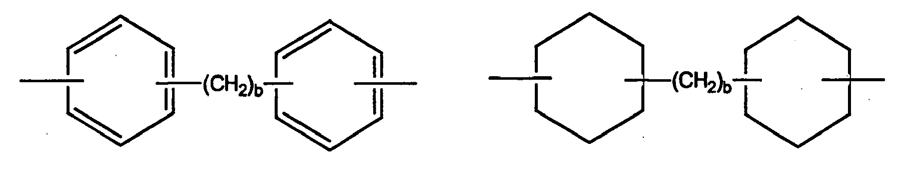 dans lesquelles b est un nombre entier compris entre 0 et 3, et c un nombre entier compris entre 1 et 20, de préférence compris entre 2 et 12.

En particulier, le radical R est choisi parmi les radicaux hexaméthylène, 4,4'-biphénylèneméthane, 2,4 et/ou 2,6-tolylène, 1,5-naphtylène, p-phénylène, méthylène-4,4bis-cyclohexyle et le radical divalent dérivé de l'isophorone.

Selon la présente invention, les polyuréthanes fixant peuvent comporter des greffons silicones et des silicones à greffons hydrocarbonés.

Un polyuréthane utilisé dans la présente invention peut avantageusement comprendre en outre au moins une séquence polysiloxane et son motif répétitif de base répond par exemple à la formule générale (IX):

-X'-P-X'-CO-NH-R-NH-CO- (IX)

dans laquelle :
- P est un segment polysiloxanique,
- X' représente O et/ou NH, et
- R est un radical divalent choisi parmi les radicaux alkylènes ramifiés ou non de type aromatique en C₆-C₂₀, aliphatique en C₁₋C₂₀, de préférence en C₁-C₆, cycloaliphatique en C₁-C₂₀, de préférence en C₁-C₆, ces radicaux étant non substitués ou substitués par un ou plusieurs groupements halogène, alcoxy en C₁-C₄, aryle en C₁-C₃₀, en particulier phényle.

Le radical R est avantageusement choisi parmi les radicaux répondant aux formules suivantes : 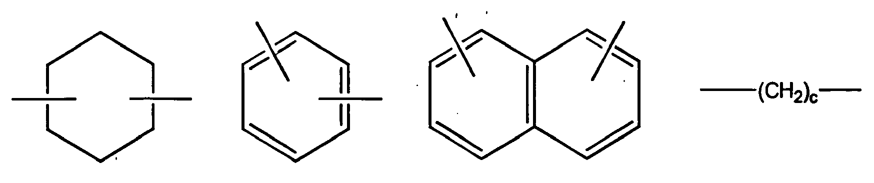 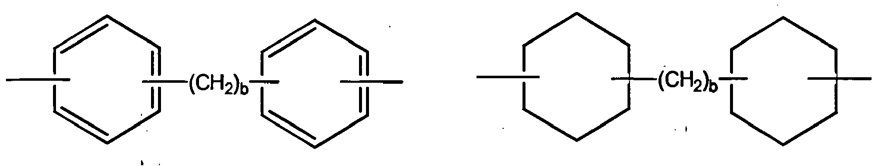 dans lesquelles b est un nombre entier compris entre 0 et 3, et c un nombre entier compris entre 1 et 20, de préférence compris entre 2 et 12.

En particulier, le radical R est choisi parmi les radicaux hexaméthylène, 4,4'-biphénylèneméthane, 2,4-et/ou 2,6-tolylène, 1,5-naphtylène, p-phénylène, méthylène-4,4bis-cyclohexyle et le radical divalent dérivé de l'isophorone.

Avantageusement, le segment polysiloxanique P répond à la formule générale (X) ci-après : 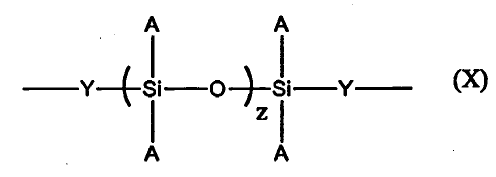 dans laquelle :
- les groupes A, qui peuvent être identiques ou différents, sont choisis parmi d'une part les groupes hydrocarbonés monovalents en C₁ à C₂₀, substantiellement exempts d'insaturation éthylénique et, d'autre part, les groupes aromatiques,
- Y représente un groupe hydrocarboné divalent, et
- Z représente un nombre entier, choisi de telle sorte que la masse moléculaire moyenne du segment polysiloxane soit comprise entre 300 et 10 000.

En général, le groupe divalent Y est choisi parmi les groupes alkylène de formule -(CH₂)ₐ-, dans laquelle a représente un nombre entier pouvant être compris entre 1 et 10.

Les groupes A peuvent être choisis parmi les groupes alkyle en C₁₋C₁₈, en particulier les groupes méthyle, éthyle, propyle, isopropyle, butyle, pentyle, hexyle, octyle, décyle, dodécyle et octadécyle ; les groupes cycloalkyle, en particulier le groupe cyclohexyle; les groupes aryle, notamment phényle et naphtyle : les groupes arylalkyle, notamment benzyle et phényléthyle, ainsi que les groupes tolyle et xylyle.

A titre d'exemples de polyuréthane fixant, on peut notamment citer le copolymère acide diméthylolpropionique/isophoronediisocyanate/néopentylglycol/polyesterdiols (connu aussi sous le nom de polyuréthane-1, appellation INCI) vendu sous la marque Luviset® PUR par la société BASF, le copolymère acide diméthylol-propionique/isophoronediisocyanate/néopentylglycol/polyesterdiols/dia-mine siliconée (connu aussi sous le nom de polyuréthane-6, appellation INCI) vendu sous la marque Luviset® Si PUR A par la société BASF.

On peut également utiliser les polymères à groupements sulfoisophtalates, tels que les polymères AQ55 et AQ48 commercialisés par la société EASTMAN.

Selon l'invention, les polymères anioniques sont de préférence choisis parmi les copolymères d'acide acrylique tels que le terpolymère acide acrylique/acrylate d'éthyle/N-tertiobutylacrylamide vendu sous la dénomination ULTRAHOLD STRONG® par la société BASF, les copolymères acide méthacrylique/acrylate d'éthyle, notamment en dispersion aqueuse, tels que les LUVIFLEX SOFT et LUVIMER MAE commercialisés par la société BASF. Les copolymères dérivés d'acide crotonique tels que les terpolymères acétate de vinyle/tertio-butylbenzoate de vinyle/acide crotonique et les terpolymères acide crotonique/acétate de vinyle/néododécanoate de vinyle vendus sous la dénomination Résine 28-29-30 par la société NATIONAL STARCH, les polymères dérivés d'acides ou d'anhydrides maléique, fumarique, itaconique avec des esters vinyliques, des éthers vinyliques, des halogénures vinyliques, des dérivés phénylvinyliques, l'acide acrylique et ses esters tels que le copolymère méthylvinyléther/anhydride maléïque monoestérifié vendu sous la dénomination GANTREZ® ES 425 par la société ISP, le LUVISET SI PUR, le MEXOMERE PW, les polyuréthanes anioniques élastomères ou non, les polymères à groupements sulfoisophtalates, les polymères siliconés greffés anioniques, ainsi que l'AMERHOLD DR 25, le VS 80.

Les polymères amphotères utilisables conformément à l'invention peuvent être choisis parmi les polymères comportant des motifs B et C répartis statistiquement dans la chaîne polymère où B désigne un motif dérivant d'un monomère comportant au moins un atome d'azote basique et C désigne un motif dérivant d'un monomère acide comportant un ou plusieurs groupements carboxyliques ou sulfoniques ou bien B et C peuvent désigner des groupements dérivant de monomères zwittérioniques de carboxybétaïnes ou de sulfobétaïnes ; B et C peuvent également désigner une chaîne polymère cationique comportant des groupements amine primaire, secondaire, tertiaire ou quaternaire, dans laquelle au moins l'un des groupements amine porte un groupement carboxylique ou sulfonique relié par l'intermédiaire d'un groupe hydrocarboné, ou bien B et C font partie d'une chaîne d'un polymère à motif éthylène-dicarboxylique dont l'un des groupements carboxyliques a été amené à réagir avec une polyamine comportant un ou plusieurs groupements amine primaire ou secondaire.

Les polymères amphotères répondant à la définition donnée ci-dessus que l'on préfère plus particulièrement, sont choisis parmi les polymères suivants :
1) les polymères résultant de la copolymérisation d'un monomère dérivé d'un composé vinylique portant un groupement carboxylique tel que plus particulièrement l'acide acrylique, l'acide méthacrylique, l'acide maléique, l'acide alpha-chloracrylique, et d'un monomère basique dérivé d'un composé vinylique substitué contenant au moins un atome basique tel que plus particulièrement les dialkylaminoalkylméthacrylate et acrylate, les dialkylaminoalkyl-méthacrylamide et acrylamide. De tels composés sont décrits dans le brevet américain n° 3 836 537.
   Le composé vinylique peut être également un sel de dialkyldiallylammonium tel que le chlorure de diéthyldiallyl-ammonium.
(2) les polymères comportant des motifs dérivant :
   a) d'au moins un monomère choisi parmi les acrylamides ou les méthacrylamides substitués sur l'azote par un groupe alkyle,
   b) d'au moins un comonomère acide contenant un ou plusieurs groupements carboxyliques réactifs, et
      a) d'au moins un comonomère basique tel que des esters à substituants amine primaire, secondaire, tertiaire et quaternaire des acides acrylique et méthacrylique, et le produit de quaternisation du méthacrylate de diméthylaminoéthyle avec le sulfate de diméthyle ou de diéthyle.
         Les acrylamides ou méthacrylamides N-substitués plus particulièrement préférés selon l'invention sont les groupements dont les groupes alkyle contiennent de 2 à 12 atomes de carbone et plus particulièrement le N-éthylacrylamide, le N-tertiobutylacrylamide, le N-tertiooctylacrylamide, le N-octylacrylamide, le N-décylacrylamide, le N-dodécylacrylamide ainsi que les méthacrylamides correspondants.
         Les comonomères acides sont choisis plus particulièrement parmi les acides acrylique, méthacrylique, crotonique, itaconique, maléique, fumarique ainsi que les monoesters d'alkyle ayant 1 à 4 atomes de carbone des acides
         ou des anhydrides maléique ou fumarique. Les comonomères basiques préférés sont des méthacrylates d'aminoéthyle, de butylaminoéthyle, de N,N'-diméthylaminoéthyle, de N-tertio-butylaminoéthyle. On utilise particulièrement les copolymères dont la dénomination CTFA (4ème Ed, 1991) est Octylacrylamide/ acrylates/butylaminoethylmethacrylate copolymer tels que les produits vendus sous la dénomination AMPHOMER® ou LOVOCRYL® 47 par la société NATIONAL STARCH.
(3) les polyaminoamides réticulés et alkylés partiellement ou totalement dérivant de polyaminoamides de formule générale : 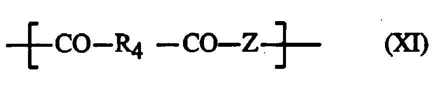 dans laquelle R₄ représente un groupe divalent dérivé d'un acide dicarboxylique saturé, d'un acide aliphatique mono ou dicarboxylique à double liaison éthylénique, d'un ester d'un alcanol inférieur ayant 1 à 6 atomes de carbone de ces acides ou d'un groupe dérivant de l'addition de l'un quelconque desdits acides avec une amine bis primaire ou bis dérivé secondaire, et Z désigne un groupe d'une polyalkylène-polyamine bis-primaire, mono ou bis-secondaire et représente de préférence :
   a) dans les proportions de 60 à 100 % en moles, le groupe 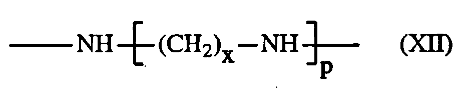 où x=2 et p=2 ou 3, ou bien x=3 et p=2
      ce groupe dérivant de la diéthylène-triamine, de la triéthylène-tétraamine ou de la dipropylène-triamine;
   b) dans les proportions de 0 à 40 % en moles, le groupe (XII) ci-dessus, dans lequel x=2 et p=1 et qui dérive de l'éthylènediamine, ou le groupe dérivant de la pipérazine 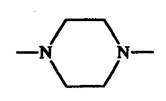
   c) dans les proportions de 0 à 20 % en moles, le groupe
      - NH-(CH₂)₆-NH- dérivant de l'hexaméthylènediamine, ces polyaminoamines étant réticulées par addition d'un agent réticulant bifonctionnel choisi parmi les épihalohydrines, les diépoxydes, les dianhydrides, les dérivés bis insaturés, au moyen de 0,025 à 0,35 mole d'agent réticulant par groupement amine du polyaminoamide et alcoylés par action d'acide acrylique, d'acide chloracétique ou d'une alcane-sultone ou de leurs sels.
      Les acides carboxyliques saturés sont choisis de préférence parmi les acides ayant 6 à 10 atomes de carbone tels que les acides adipique, 2,2,4-triméthyladipique et 2,4,4-triméthyladipique, téréphtalique, les acides à double liaison éthylénique comme, par exemple, les acides acrylique, méthacrylique, itaconique. Les alcane-sultones utilisées dans l'alcoylation sont de préférence la propane- ou la butane-sultone, les sels des agents d'alcoylation sont de préférence les sels de sodium ou de potassium.
(4) les polymères comportant des motifs zwittérioniques de formule : 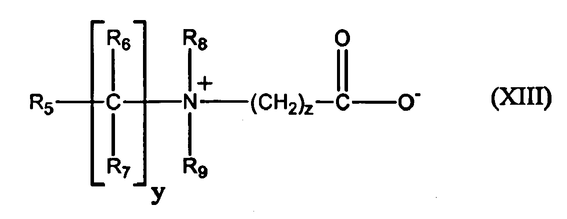 dans laquelle R₅ désigne un groupement insaturé polymérisable tel qu'un groupement acrylate, méthacrylate, acrylamide ou méthacrylamide, y et z représentent chacun un nombre entier de 1 à 3, R₆ et R₇ représentent un atome d'hydrogène, un groupement méthyle, éthyle ou propyle, R₈ et R₉ représentent un atome d'hydrogène ou un groupe alkyle de telle façon que la somme des atomes de carbone dans R₁₀ et R₁₁ ne dépasse pas 10.
   Les polymères comprenant de tels motifs peuvent également comporter des motifs dérivés de monomères non zwittérioniques tels que l'acrylate ou le méthacrylate de diméthyl- ou diéthylaminoéthyle, ou des acrylates ou méthacrylates d'alkyle, des acrylamides ou méthacrylamides, ou l'acétate de vinyle.
(5) les polymères dérivés du chitosane comportant des motifs monomères répondant aux formules suivantes: 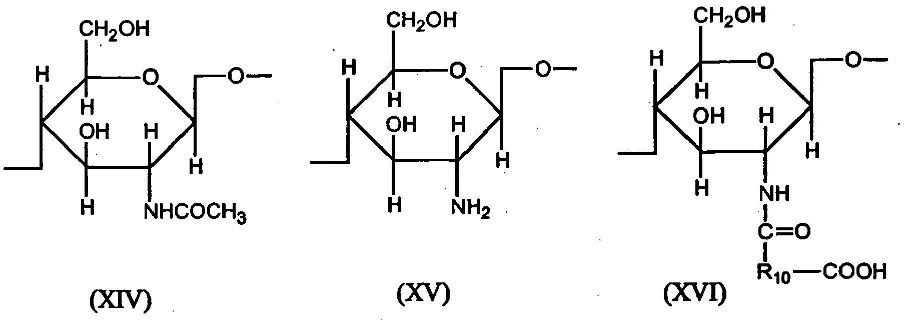 le motif (XIV) étant présent dans des proportions comprises entre 0 et 30 %, le motif (XV) dans des proportions comprises entre 5 et 50 % et le motif (XVI) dans des proportions comprises entre 30 et 90 %, étant entendu que dans ce motif F, R₁₀ représente un groupe de formule: 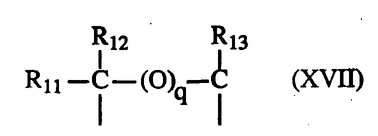 dans laquelle si q=0, R₁₁, R₁₂ et R₁₃, identiques ou différents, représentent chacun un atome d'hydrogène, un reste méthyle, hydroxyle, acétoxy ou amino, un reste monoalkylamine ou un reste dialkylamine éventuellement interrompus par un ou plusieurs atomes d'azote et/ou éventuellement substitués par un ou plusieurs groupes amine, hydroxyle, carboxyle, alkylthio, sulfonique, un reste alkylthio dont le groupe alkyle porte un reste amino, l'un au moins des groupes R₁₇, R₁₈ et R₁₉ étant dans ce cas un atome d'hydrogène ;
   ou si q=1, R₁₁, R₁₂ et R₁₃ représentent chacun un atome d'hydrogène, ainsi que les sels formés par ces composés avec des bases ou des acides.
(6) Les polymères dérivés de la N-carboxyalkylation du chitosane.
(7) Les polymères répondant à la formule générale (XVIII) décrits par exemple, dans le brevet français 1 400 366: 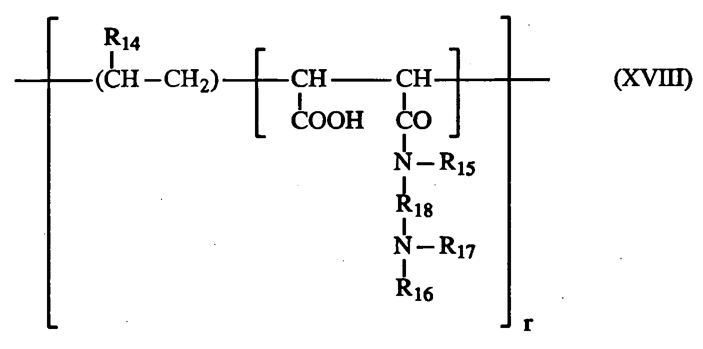 dans laquelle R₁₄ représente un atome d'hydrogène, un groupe CH₃O, CH₃CH₂O, phényle, R₁₅ désigne l'hydrogène ou un groupe alkyle inférieur tel que méthyle, éthyle, R₁₆ désigne l'hydrogène ou un groupe alkyle inférieur tel que méthyle, éthyle, R₁₇ désigne un groupe alkyle inférieur tel que méthyle, éthyle ou un groupe répondant à la formule: -R₁₈-N(R₁₆)₂, R₁₈ représentant un groupement -CH₂-CH₂-, -CH₂-CH₂-CH₂-, -CH₂-CH(CH₃)-, R₁₆ ayant les significations mentionnées ci-dessus,
   ainsi que les homologues supérieurs de ces groupes et contenant jusqu'à 6 atomes de carbone.
(8) Des polymères amphotères du type -D-X-D-X- choisis parmi:
   a) les polymères obtenus par action de l'acide chloracétique ou le chloracétate de sodium sur les composés comportant au moins un motif de formule:

      -D-X-D-X-D- (XIX)

      où D désigne un groupe 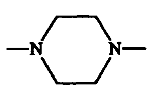 et X désigne le symbole E ou E', E ou E', identiques ou différents, désignent un groupe bivalent qui est un groupe alkylène à chaîne droite ou ramifiée comportant jusqu'à 7 atomes de carbone dans la chaîne principale non substituée ou substituée par des groupements hydroxyle, et pouvant comporter en outre des atomes d'oxygène, d'azote, de soufre, 1 à 3 cycles aromatiques et/ou hétérocycliques ; les atomes d'oxygène, d'azote et de soufre étant présents sous forme de groupements éther, thioéther, sulfoxyde, sulfone, sulfonium, alkylamine, alcénylamine, des groupements hydroxyle, benzylamine, oxyde d'amine, ammonium quaternaire, amide, imide, alcool, ester et/ou uréthanne.
   b) Les polymères de formule :

      -D-X-D-X- (XX)

      où D désigne un groupe 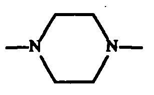 et X désigne le symbole E ou E' et au moins une fois E'; E ayant la signification indiquée ci-dessus et E' est un groupe bivalent qui est un groupe alkylène à chaîne droite ou ramifiée ayant jusqu'à 7 atomes de carbone dans la chaîne principale, substitué ou non par un ou plusieurs groupes hydroxyle et comportant un ou plusieurs atomes d'azote, l'atome d'azote étant substitué par une chaîne alkyle interrompue éventuellement par un atome d'oxygène et comportant obligatoirement une ou plusieurs fonctions carboxyle ou une
      ou plusieurs fonctions hydroxyle et bétaïnisées par réaction avec l'acide chloracétique ou du chloracétate de soude.
(9) Les copolymères alkyl(C₁-C₅)vinyléther/anhydride maléique modifié partiellement par semiamidification avec une N,N-dialkylaminoalkylamine telle que la N,N-diméthylaminopropyl-amine ou par semiestérification avec une N,N-dialcanolamine Ces copolymères peuvent également comporter d'autres comonomères vinyliques tels que le vinylcaprolactame.

Selon un mode de réalisation préféré de l'invention, les polymères amphotères fixants utilisables dans le procédé selon l'invention peuvent être choisis parmi les copolymères à blocs, ramifiés, comprenant :
(a) des motifs non ioniques dérivés d'au moins un monomère choisi parmi les (méth)acrylates d'alkyle en C₁-C₂₀, les N-mono-(alkyle en C₂-C₁₂)-(méth)acrylamides et les N,N-di-(alkyle en C₂-C₁₂)-(méth)acrylamide,
(b) des motifs anioniques dérivés d'au moins un monomère choisi parmi l'acide acrylique et l'acide méthacrylique, et
(c) des motifs polyfonctionnels dérivés d'au moins un monomère comportant au moins deux groupes fonctionnels insaturés polymérisables, et ayant de préférence une structure constituée de blocs hydrophobes sur lesquels sont fixés, par l'intermédiaire des motifs polyfonctionnels (c), plusieurs blocs plus hydrophiles.

De préférence, les polymères amphotères présentent au moins deux températures de transition vitreuse (Tg) dont au moins une est supérieure à 20 °C et l'autre est inférieure à 20°C.

Les polymères amphotères préférés sont les polymères comportant des motifs dérivant :
a) d'au moins un monomère choisi parmi les acrylamides ou les méthacrylamides substitués sur l'azote par un groupe alkyle,
b) d'au moins un comonomère acide contenant un ou plusieurs groupements carboxyliques réactifs, et
c) d'au moins un comonomère basique tel que des esters à substituants amine primaire, secondaire, tertiaire et quaternaire des acides acrylique et méthacrylique, et le produit de quaternisation du méthacrylate de diméthylaminoéthyle avec le sulfate de diméthyle ou de diéthyle.

On peut citer en particulier les polymères vendus sous la dénomination AMPHOMER par la société NATIONAL STARCH.

Généralement, le ou les polymères fixants anioniques et/ou amphotères représentent de 0,1 à 50 %, de préférence de 1 à 30 %, en poids du poids total de la première composition.

Comme expliqué précédemment, une deuxième composition est appliquée sur les cheveux, avant ou après l'application de la première composition comprenant un ou plusieurs polymères fixants solubilisés.

La deuxième composition comprend, dans un milieu cosmétiquement acceptable, un ou plusieurs sels minéraux et/ou organiques.

L'anion ou les anions du ou des sels organiques et/ou minéraux peuvent être des anions organiques monovalents ou polyvalents, des anions minéraux monovalents ou polyvalents.

Ainsi, l'anion ou les anions du ou des sels organiques et/ou minéraux est en général choisi parmi les nitrates, les sulfates, les carbonates, les halogénures et en particulier les chlorures, les bromates, les phosphates et les sulfonates.

Par ailleurs, le ou les sels minéraux et/ou organiques peuvent être un sel d'ammonium ou d'un métal alcalin, alcalino-terreux ou de transition.

En particulier, le métal peut être choisi parmi le cuivre, l'argent, l'or, le magnésium, le sodium, le potassium, le calcium, le fer, le platine, le titane, le zinc et leurs alliages.

Ainsi, par exemple, le ou les sels organiques et/ou minéraux peuvent être choisis parmi NaCl, MgSO₄, ZnSO₄, ZnCl₂, MgCl₂, Na₂SO₄, Na₂CO₃, NH₄Cl, AgNO₃, Ag₂SO₄, Ag₂CO₃, AgCl et leurs mélanges.

De préférence, les sels utilisés sont NaCl et MgSO₄.

Tous ces sels peuvent éventuellement être hydratés.

Le ou les sels minéraux et/ou organiques représentent généralement de 0,15 à 30 %, de préférence de 0,5 à 20 %, en poids du poids total de la deuxième composition.

Comme expliqué précédemment, le ou les polymères fixants anioniques et/ou amphotères et le ou les sels minéraux ou organiques sont respectivement présents dans la première et la deuxième composition, à chaque fois dans un milieu cosmétiquement acceptable.

Le milieu cosmétiquement acceptable de la première et de la deuxième composition est généralement choisi parmi l'eau, les alcools en C₁-C₆, de préférence les alcanols tels que l'éthanol, le propanol et l'isopropanol, les alcanediols tels que l'éthylèneglycol, le propylène glycol et le pentanediol, l'alcool benzylique, les alcanes en C₅-C₁₀, l'acétone, la méthyléthylcétone, l'acétate de méthyle, l'acétate de butyle, l'acétate d'éthyle, le diméthoxyéthane, le diéthoxyéthane et leurs mélanges.

La première et/ou la deuxième composition peuvent en outre comprendre des additifs cosmétiques usuels choisis notamment parmi les agents épaississants, les adoucissants, les agents anti-mousse, les filtres solaires, les agents hydratants, les agents alcalinisants, les colorants, les pigments, les parfums, les conservateurs, les tensioactifs anioniques, cationiques, non ioniques ou amphotères, les polymères non fixants, les silicones volatiles ou non, les huiles végétales, animales, minérales ou de synthèse, les protéines, les vitamines, les polyols et leurs mélanges.

La première et la deuxième compositions peuvent être des solutions homogènes, des suspensions, des émulsions eau-dans-huile (E/H), des émulsions huile-dans-eau (H/E) ou des émulsions multiples, toutes ayant une consistance fluide, plus ou moins épaissie, ou bien gélifiée.

Le pH de la première et de la deuxième compositions est généralement compris entre 4 et 10, de préférence 6 à 10 pour la première composition et 4 à 8 pour la deuxième composition.

Le réglage du pH de ces compositions peut être obtenu à l'aide d'un agent alcalin, tel que, par exemple, l'ammoniaque, la monoéthanolamine, la diéthanolamine, la triéthanolamine, la 1,3-propanediamine, un hydroxyde alcalin, tel que le 2-amino-2-méthyl-1-propanol, ou bien à l'aide d'un agent acidifiant tel que par exemple l'acide chlorhydrique, l'acide acétique, l'acide lactique, l'acide oxalique ou l'acide borique.

La mise en forme des cheveux peut être effectuée à la main, avec un peigne, une brosse ou des rouleaux de 2 à 30 mm de diamètre.

Comme expliqué précédemment, le polymère fixant est présent sous forme solubilisée dans la première composition.

L'invention est illustrée par les exemples suivants.

### Exemples

On met en oeuvre le procédé selon l'invention en utilisant une première composition et une deuxième composition.

La première et la deuxième composition présentent les formulations suivantes (MA désigne matières actives) :

### Première composition :

| | |
|---|---|
| LUVISET Si-P.U.R.A (BASF) | 15 g MA |
| 2-amino-2-méthyl-1-propanol | qs pH 9 |
| Ethanol | 10 g |
| Eau déminéralisée | qsp 100 g |

### Deuxième composition :

| | |
|---|---|
| Chlorure d'ammonium | 15 g |
| Eau déminéralisée | qsp 100 g pH = 5,5 |

La première composition est appliquée sur une partie de la chevelure concernée. Les cheveux sont alors mis en forme à la main. Puis la deuxième composition est appliquée. On laisse poser 5 minutes à température ambiante, puis on rince les cheveux.

La partie traitée selon l'invention présente une très bonne tenue de coiffure, rémanente aux shampooings. Les cheveux sont doux et le toucher très naturel.

## Revendications

1. Procédé de mise en forme des cheveux, **caractérisé en ce qu'**il comprend les étapes suivantes :
(a) application sur les cheveux d'une première composition comprenant, dans un milieu cosmétiquement acceptable, un ou plusieurs polymères fixants solubilisés, le ou lesdits polymères fixants consistant en un ou plusieurs polymères anioniques et/ou amphotères, cette application étant éventuellement suivie d'un temps de pose de ladite première composition,
(b) application sur les cheveux d'une deuxième composition comprenant, dans un milieu cosmétiquement acceptable, au moins 0,15%, en poids du poids total de ladite deuxième composition, d'un ou plusieurs sels minéraux et/ou organiques, cette application étant éventuellement suivie d'un temps de pose de ladite deuxième composition,
l'étape b) étant effectuée avant ou après l'étape a), puis
(c) rinçage des cheveux,
une étape de mise en forme des cheveux s'effectuant soit après l'application de la première composition de l'étape a), soit après l'application de la deuxième composition de l'étape b), et avant l'étape de rinçage c),
ledit procédé ne comprenant pas d'étape d'ouverture des liaisons disulfures de la kératine des cheveux par une composition réductrice, ni d'étape de reconstitution desdites liaisons disulfures par une composition oxydante.

2. Procédé selon la revendication 1, **caractérisé en ce que** le ou les polymères fixants sont choisis parmi les polymères amphotères choisis parmi les copolymères comportant des motifs dérivant :
a) d'au moins un monomère choisi parmi les acrylamides ou les méthacrylamides substitués sur l'azote par un groupe alkyle,
b) d'au moins un comonomère acide contenant un ou plusieurs groupements carboxyliques réactifs, et
c) d'au moins un comonomère basique tel que des esters à substituants amine primaire, secondaire, tertiaire et quaternaire des acides acrylique et méthacrylique, et le produit de quatemisation du méthacrylate de diméthylaminoéthyle avec le sulfate de diméthyle ou de diéthyle.

3. Procédé selon la revendication 1, **caractérisé en ce que** les polymères anioniques sont choisis parmi les copolymères d'acide acrylique, les copolymères dérivés d'acide crotonique, les polymères dérivés d'acides ou d'anhydrides maléique, fumarique, itaconique avec des esters vinyliques, des éthers vinyliques, des halogénures vinyliques, des dérivés phénylvinyliques, l'acide acrylique et ses esters, les polyuréthanes anioniques élastomères ou non, les polymères à groupements sulfoisophtalates et les polymères siliconés greffés anioniques.

4. Procédé selon l'une quelconque des revendications précédentes **caractérisé en ce que** le ou les polymères fixants représentent de 0,1 à 50 %, de préférence de 1 à 30 %, en poids du poids total de la première composition.

5. Procédé selon l'une quelconque des revendications précédentes, **caractérisé en ce que** l'anion ou les anions du ou des sels minéraux ou organiques sont des anions organiques mono ou polyvalents, des anions minéraux monovalents ou polyvalents ou des anions minéraux soufrés.

6. Procédé selon la revendication 5, **caractérisé en ce que** le ou les anions sont choisis parmi les nitrates, les sulfates, les carbonates, les halogénures, en particulier les chlorures, les bromates, les phosphates et les sulfonates.

7. Procédé selon l'une quelconque des revendications précédentes **caractérisé en ce que** le ou les sels minéraux ou organiques sont des sels d'ammonium ou d'un métal alcalin, alcalino-terreux ou de transition.

8. Procédé selon la revendication 7, **caractérisé en ce que** le métal est choisi parmi le cuivre, l'argent, l'or, le fer, le platine, le sodium, le potassium, le calcium, le magnésium, le titane, le zinc et leurs alliages.

9. Procédé selon l'une quelconque des revendications 5 à 8, **caractérisé en ce que** le ou les sels organiques ou minéraux sont choisis parmi NaCl, MgSO₄, ZnSO₄, ZnCl₂, MgCl₂, Na₂SO₄, Na₂CO₃, NH₄Cl, AgNO₃, Ag₂SO₄, Ag₂CO₃, AgCl et leurs mélanges.

10. Procédé selon l'une quelconque des revendications précédentes, **caractérisé en ce que** le ou les sels minéraux et/ou organiques représentent de 0,15 à 30 %, de préférence de 0,5 à 20 %, en poids du poids total de la deuxième composition.

## Claims

1. A hair shaping method **characterized in that** it comprises the following steps consisting in:
(a) applying onto the hair a first composition comprising in a cosmetically acceptable medium, one or more solubilized fixing polymer(s), said fixing polymer(s) consisting in one or more anionic and/or amphoteric polymer(s), this application being optionally followed by a resting time for said first composition,
(b) applying onto the hair a second composition comprising in a cosmetically acceptable medium at least 0.15% by weight as related to the total weight of said second composition of one or more mineral and/or organic salt(s), such application being optionally followed by a resting time for said second composition,
where step b) is conducted prior to or after step a), then
(c) rinsing the hair,
a hair shaping step being carried out either after applying the first composition of step a), or after applying the second composition of step b) and prior to the rinsing step c),
said method neither comprising any step consisting in opening the hair keratin disulfide bonds by means of a reducing composition, nor any step consisting in reforming said disulfide bonds by means of an oxidizing composition.

2. A method according to claim 1, **characterized in that** the fixing polymer(s) is or are selected from amphoteric polymers chosen from copolymers comprising units derived from:
a) at least one monomer selected from acrylamides or methacrylamides, substituted on the nitrogen with an alkyl group,
b) at least one acidic comonomer comprising one or more reactive carboxylic moieties, and
c) at least one basic comonomer, such as primary, secondary, tertiary and quaternary amine substituted esters of acrylic and methacrylic acids, and the quaternization product of dimethyl aminoethyl methacrylate with dimethyl or diethyl sulfate.

3. Method according to claim 1, **characterized in that** the anionic polymers are selected from copolymers of acrylic acid, copolymers derived from crotonic acid, polymers derived from maleic, fumaric, itaconic acids or anhydrides with vinyl esters, vinyl ethers, vinyl halides, phenyl vinyl derivatives, acrylic acid and esters thereof, anionic polyurethanes elastomer or non elastomer, sulfoisophthalate moiety polymers and anionic silicone graft polymers.

4. A method according to any one of preceding claims, **characterized in that** the fixing polymer(s) represent(s) from 0.1 to 50%, preferably from 1 to 30% by weight, as related to the first composition total weight.

5. A method according to any one of preceding claims, **characterized in that** the anion(s) of the organic and/or mineral salt(s) are monovalent or polyvalent organic anions, monovalent or polyvalent mineral anions, or mineral sulfur anions.

6. A method according to claim 5, **characterized in that** the anion(s) is or are selected from nitrates, sulfates, carbonates, halides, in particular chlorides, bromates, phosphates and sulfonates.

7. A method according to any one of preceding claims, **characterized in that** the mineral and/or organic salt(s) are ammonium salts or alkaline metal salts, alkaline earth metal salts or transition metal salts.

8. A method according to claim 7, **characterized in that** the metal is selected from copper, silver, gold, iron, platinum, sodium, potassium, calcium, magnesium, titanium, zinc and alloys thereof.

9. A method according to any one of claims 5 to 8, **characterized in that** the organic and/or mineral salt(s) is or are selected from NaCl, MgSO₄, ZnSO₄, ZnCl₂, MgCl₂, Na₂SO₄, Na₂CO₄, NH₄Cl, AgNO₃, Ag₂SO₄, Ag₂CO₃, AgCl and their mixtures.

10. A method according to any one of preceding claims, **characterized in that** the mineral and/or organic salt(s) represent(s) from 0.15 to 30%, preferably from 0.5 to 20% by weight, as related to the second composition total weight.

## Patentansprüche

1. Verfahren zum Stylen ("procédé de mise en forme") der Haare, **dadurch gekennzeichnet, dass** es die folgenden Stufen umfasst:
(a) Auftragen einer ersten Zusammensetzung auf die Haare, die, in einem kosmetisch verträglichen Medium, ein oder mehrere solubilisierte(s) fixierende(s) Polymer(e) umfasst, wobei das fixierende Polymer oder die fixierenden Polymere aus einem oder mehreren anionischen und/oder amphoteren Polymer(en) besteht/bestehen, wobei auf diese Auftragung gegebenenfalls eine Einwirkzeit der ersten Zusammensetzung folgt;
(b) Auftragen einer zweiten Zusammensetzung auf die Haare, die, in einem kosmetisch verträglichen Medium, wenigstens 0,15 Gew.-%, bezogen auf das Gesamtgewicht der zweiten Zusammensetzung, eines oder mehrerer mineralischer und/oder organischer Salze umfasst, wobei auf diese Auftragung gegebenenfalls eine Einwirkzeit der zweiten Zusammensetzung folgt,
wobei die Stufe b) vor oder nach der Stufe a) durchgeführt wird, danach
(c) Ausspülen der Haare,
wobei eine Stufe des Stylens ("mise en forme") der Haare entweder nach Auftragung der ersten Zusammensetzung der Stufe a) oder nach Auftragung der zweiten Zusammensetzung der Stufe b) und vor der Stufe des Ausspülens c) durchgeführt wird,
wobei das Verfahren weder eine Stufe der Öffnung der Disulfidbindungen des Keratins der Haare durch eine reduzierende Zusammensetzung noch eine Stufe der Rekonstitution der Disulfidbindungen durch eine oxidierende Zusammensetzung umfasst.

2. Verfahren gemäß Anspruch 1, **dadurch gekennzeichnet, dass** das fixierende Polymer oder die fixierenden Polymere ausgewählt ist/sind aus den amphoteren Polymeren, ausgewählt aus den Copolymeren, die Repetiereinheiten umfassen, abgeleitet von:
a) wenigstens einem Monomer, ausgewählt aus Acrylamiden oder Methacrylamiden, die am Stickstoff mit einer Alkylgruppe substituiert sind,
b) wenigstens einem sauren Comonomer, das eine oder mehrere reaktive Carboxylgruppen enthält, und
c) wenigstens einem basischen Comonomer wie Estern mit primären, sekundären, tertiären oder quaternären Amin-Substituenten von Acryl- und Methacrylsäuren und das Quaternisierungsprodukt von Dimethylaminoethylmethacrylat mit Dimethyl- oder Diethylsulfat.

3. Verfahren gemäß Anspruch 1, **dadurch gekennzeichnet, dass** die anionischen Polymere ausgewählt sind aus Copolymeren von Acrylsäure, Copolymeren, die von Crotonsäure abgeleitet sind, Polymeren, die von Malein-, Fumar-, Itaconsäure oder -anhydrid abgeleitet sind, mit Vinylestern, Vinylethern, Vinylhalogeniden, Phenylvinylderivaten, Acrylsäure und ihren Estern, anionischen Polyurethanen, die Elastomere sind oder nicht, Polymeren mit Sulfoisophtalat-Gruppierungen und anionischen Pfropfsilikonpolymeren.

4. Verfahren gemäß einem der vorangehenden Ansprüche, **dadurch gekennzeichnet, dass** das fixierende Polymer oder die fixierenden Polymere 0,1 bis 50 Gew.-%, vorzugsweise 1 bis 30 Gew.-%, des Gesamtgewichts der ersten Zusammensetzung darstellt/darstellen.

5. Verfahren gemäß einem der vorangehenden Ansprüche, **dadurch gekennzeichnet, dass** das Anion oder die Anionen des oder der mineralischen oder organischen Salze(s) mono- oder polyvalente organische Anionen, monovalente oder polyvalente mineralische Anionen oder mineralische schwefelhaltige Anionen ist/sind.

6. Verfahren gemäß Anspruch 5, **dadurch gekennzeichnet, dass** das Anion oder die Anionen ausgewählt ist/sind aus Nitraten, Sulfaten, Carbonaten, Halogeniden, insbesondere Chloriden, Bromaten, Phosphaten und Sulfonaten.

7. Verfahren gemäß einem der vorangehenden Ansprüche, **dadurch gekennzeichnet, dass** das mineralische oder organische Salz oder die mineralischen oder organischen Salze Salze von Ammonium oder einem Alkalimetall, Erdalkalimetall oder Übergangsmetall sind.

8. Verfahren gemäß Anspruch 7, **dadurch gekennzeichnet, dass** das Metall ausgewählt ist aus Kupfer, Silber, Gold, Eisen, Platin, Natrium, Kalium, Calcium, Magnesium, Titan, Zink und deren Legierungen.

9. Verfahren gemäß einem der Ansprüche 5 bis 8, **dadurch gekennzeichnet, dass** das organische oder mineralische Salz oder die organischen oder mineralischen Salze ausgewählt sind aus NaCl, MgSO₄, ZnSO₄, ZnCl₂, MgCl₂, Na₂SO₄, Na₂CO₃, NH₄Cl, AgNO₃, Ag₂SO₄, Ag₂CO₃, AgCl und deren Gemischen.

10. Verfahren gemäß einem der vorangehenden Ansprüche, **dadurch gekennzeichnet, dass** das mineralische und/oder organische Salz oder die mineralischen und/oder organischen Salze 0,15 bis 30 Gew.-%, vorzugsweise 0,5 bis 20 Gew.-%, bezogen auf das Gesamtgewicht der zweiten Zusammensetzung, darstellt/darstellen.
